# EUROPÄISCHE PATENTANMELDUNG

(11) **EP 1 046 392 A2**
(43) Veröffentlichungstag der Anmeldung: **25.10.2000**
(21) Anmeldenummer: 00108526.5
(22) Anmeldetag: 19.04.2000
(51) Int. Cl.: A61K 7/48, A61K 7/06

(54) **Kosmetische und dermatologische Zubereitungen für die Verbesserung der Barrierefunktion**

(30) Priorität: 24.04.1999 DE 19918750
(71) Anmelder: Beiersdorf AG, 20245 Hamburg (DE)
(72) Erfinder: Schreiner, Dr. Volker, 20259 Hamburg (DE); Sauermann, Dr. Gerhard, 24649 Wiemersdorf (DE); Schönrock, Dr. Uwe, 23866 Nahe (DE); Max, Dr. Heiner, 22529 Hamburg (DE); Sandhoff, Prof. Dr. Konrad, 53347 Alfter (DE); Döring, Thomas, 20255 Hamburg (DE)

(57) **Zusammenfassung**

Gegenstand der Erfindung ist die topische Verwendung eines Wirkstoffes oder mehrerer Wirkstoffe, ausgewählt aus der Gruppe der NO oder NO-Radikale bindenden Substanzen und der Gruppe der die NO-Synthese inhibierenden Substanzen und der Gruppe der Inhibitoren der Wirkungen von NO oder NO-Radikalen, zur Verbesserung der Funktion der Permeabilitätsbarriere der Haut und/oder der Verbesserung der Struktur oder des Wachstums der Haare.

## Beschreibung

Die vorliegende Erfindung betrifft insbesondere Wirkstoffe und topische Zubereitungen zur Pflege und Reinigung trockener und beanspruchter Haut und Altershaut sowie zur Behandlung und Prophylaxe der Hautaustrocknung und der Beanspruchung der Haut sowie jeweils deren Folgeschäden, wie sie beispielsweise bei der Behandlung mit waschaktiven Substanzen auftreten können. Folgeschäden sind beispielsweise Rissigkeit und Schuppigkeit der Haut und der Kopfhaut.

Die Haut ist das größte Organ des Menschen. Unter ihren vielen Funktionen (beispielsweise zur Wärmeregulation und als Sinnesorgan) ist die Barrierefunktion, die das Austrocknen der Haut (und damit letzlich des gesamten Organismus) verhindert, die wohl wichtigste. Gleichzeitig wirkt die Haut als Schutzeinrichtung gegen das Eindringen und die Aufnahme von außen kommender Stoffe. Bewirkt wird diese Barrierefunktion durch die Epidermis, welche als äußerste Schicht die eigentliche Schutzhülle gegenüber der Umwelt bildet. Mit etwa einem Zehntel der Gesamtdicke ist sie gleichzeitig die dünnste Schicht der Haut.

Die Epidermis ist ein stratifiziertes Gewebe, in dem die äußere Schicht, die Hornschicht (Stratum corneum), den für die Barrierefunktion bedeutenden Teil darstellt. Sie wird im Kontakt mit der Umwelt abgenutzt und befindet deshalb sich in einem ständigen Erneuerungsprozess, wobei nach außen kontinuierlich feine Schuppen abgegeben und von innen verhorntes Zell- und Lipidmaterial nachproduziert wird.

Das heute in der Fachwelt anerkannte Hautmodell von Elias (*P. M. Elias, Structure and Function of the Stratum Corneum Permeability Barrier, Drug Dev. Res. **13**, 1988, 97-105*) beschreibt die Hornschicht als Zwei-Komponenten-System, ähnlich einer Ziegelsteinmauer (Ziegelstein-Mortel-Modell) in diesem Modell entsprechen die Hornzellen (Korneozyten) den Ziegelsteinen, die komplex zusammengesetzte Lipidmembran in den Interzellularräumen entspricht dem Mörtel Dieses System stellt im wesentlichen eine physikalische Barriere gegen hydrophile Substanzen dar. kann aber aufgrund seiner engen und mehrschichtigen Struktur gleichermaßen auch von lipophilen Substanzen nur schwer passiert werden. Die besondere Struktur der Hornschicht schützt einerseits die Haut und stabilisiert andererseits ihre eigene Flexibilität durch Bindung einer definierten Wassermenge.

Auch mechanische Belastungen, wie beispielsweise Druck-, Stoß- oder Scherkräfte können in erstaunlichem Maße durch die Hornschicht allein oder im Verbund mit den tieferen Hautschichten abgefangen werden. Größere Druck-, Dreh- oder Scherkräfte werden über die Verzahnung der Epidermis mit dem Corium an tiefere Hautschichten weitergegeben.

Die Regulation des Wasser- und Feuchtigkeitsgehaltes ist eine der wichtigsten Funktionen der epidermalen Lipidmembran. Allerdings hat sie nicht nur eine Barrierewirkung gegen externe chemische und physikalische Einflüsse, sondern trägt auch zum Zusammenhalt der Hornschicht bei.

Die Lipide der Hornschicht bestehen im wesentlichen aus Ceramiden, freien Fettsäuren, Cholesterin und Cholesterinsulfat und sind über die gesamte Hornschicht verteilt. Die Zusammensetzung dieser Lipide ist für die intakte Funktion der epidermalen Barriere und damit für die Wasserundurchlässigkeit der Haut von entscheidender Bedeutung.

Bereits bei einer Reinigung der Haut mit Hilfe eines einfachen Wasserbads - ohne Zusatz von Tensiden - kommt es zunächst zu einer Quellung der Hornschicht der Haut. Der Grad dieser Quellung hängt u. a. von der Dauer des Bads und dessen Temperatur ab. Gleichzeitig werden wasserlösliche Stoffe ab- bzw. ausgewaschen, wie z.B. wasserlösliche Schmutzbestandteile, aber auch hauteigene Stoffe, die für das Wasserbindungsvermögen der Hornschicht verantwortlich sind. Durch hauteigene oberflächenaktive Stoffe werden außerdem auch Hautfette in gewissem Ausmaß gelöst und ausgewaschen. Dies bedingt nach anfänglicher Quellung eine nachfolgende Austrocknung der Haut, die durch waschaktive Zusätze noch deutlich verstärkt werden kann.

Bei gesunder Haut sind diese Vorgänge im allgemeinen belanglos, da die Schutzmechanismen der Haut solche leichten Störungen der oberen Hautschichten ohne weiteres kompensieren können. Aber bereits im Fall nichtpathologischer Abweichungen vom Normalstatus, z.B durch umweltbedingte Abnutzungsschäden bzw. Irritationen, Lichtschäden, Altershaut usw., ist der Schutzmechanismus an der Hautoberflache gestört.

Bei alter Haut beispielsweise erfolgt die regenerative Erneuerung verlangsamt, wobei insbesondere das Wasserbindungsvermögen der Hornschicht nachläßt. Sie wird deshalb inflexibel, trocken und rissig ("physiologisch" trockene Haut). Ein Barriereschaden ist die Folge. Die Haut wird anfällig für negative Umwelteinflüsse wie die Invasion von Mikroorganismen, Toxinen und Allergenen. Als Folge kann es sogar zu toxischen oder allergischen Hautreaktionen kommen.

Bei pathologisch trockener und empfindlicher Haut liegt ein Barriereschaden a priori vor. Epidermale Interzellularlipide werden offenbar fehlerhaft oder in ungenügender Menge bzw. Zusammensetzung gebildet. Die Konsequenz ist eine erhöhte Durchlässigkeit der Hornschicht und ein unzureichender Schutz der Haut vor Verlust an hygroskopischen Substanzen und Wasser.

Die Barrierewirkung der Haut kann über die Bestimmung des transepidermalen Wasserverlustes (TEWL - transepidermal water loss) quantifiziert werden. Dabei handelt es sich um die Abdunstung von Wasser aus dem Körperinneren ohne Einbeziehung des Wasserverlustes beim Schwitzen. Die Bestimmung des TEWL-Wertes hat sich als außerordentlich informativ erwiesen und kann zur Diagnose rissiger oder schrundiger Haut, zur Bestimmung der Verträglichkeit chemisch verschiedenartig aufgebauter Tenside und dergleichen mehr herangezogen werden.

Für die Schönheit und Gepflegtheit der Haut ist der Wasseranteil in der obersten Hautschicht von größter Bedeutung. Man kann ihn in einem begrenzten Umfang durch Einbringen von Feuchtigkeitsregulatoren günstig beeinflussen.

Anionische Tenside, welche im allgemeinen Bestandteile von Reinigungszubereitungen sind, können den pH-Wert in der Hornschicht langanhaltend erhöhen, was regenerative Prozesse, die der Wiederherstellung und Erneuerung der Barrierefunktion der Haut dienen, stark behindert. In diesern Fall stellt sich in der Hornschicht zwischen Regeneration und dem Verlust essentieller Substanzen durch regelmäßige Extraktion ein neuer, häufig sehr ungünstiger Gleichgewichtszustand ein, der das äußere Erscheinungsbild der Haut und die physiologische Funktionweise der Hornschicht entscheidend beeinträchtigt.

Unter Hautpflege im Sinne der vorliegenden Erfindung ist in erster Linie zu verstehen, daß die natürliche Funktion der Haut als Barriere gegen Umwelteinflüsse (z.B. Schmutz, Chemikalien, Mikroorganismen) und gegen den Verlust von körpereigenen Stoffen (z.B. Wasser, Lipide, Elektrolyte) gestärkt oder wiederhergestellt wird.

Produkte zur Pflege, Behandlung und Reinigung trockener und strapazierter Haut sind an sich bekannt. Allerdings ist ihr Beitrag zur Regeneration einer physiologisch intakten, hydratisierten und glatten Hornschicht umfangmäßig und zeitlich begrenzt.

Die Wirkung von Salben und Cremes auf die Barrierefunktion und die Hydratation der Hornschicht beruht im wesentlichen auf der Abdeckung (Okklusion) der behandelten Hautbezirke. Die Salbe oder Creme stellt sozusagen eine (zweite) künstliche Barriere dar, die den Wasserverlust der Haut verhindern soll. Entsprechend leicht kann diese physikalische Barriere - beispielsweise mit Reinigungsmitteln - wieder entfernt werden, wodurch der ursprüngliche, beeinträchtigte Zustand wieder erreicht wird. Darüber hinaus kann die Hautpflegewirkung bei regelmäßiger Behandlung nachlassen. Nach dem Absetzen der Produktanwendung kehrt die Haut sehr schnell wieder in den Zustand vor Behandlungsbeginn zurück. Bei bestimmten Produkten verschlechtert sich der Zustand der Haut unter Umständen sogar vorübergehend. Eine nachhaltige Produktwirkung wird in der Regel also nicht oder nur in einem eingeschränkten Maße erreicht.

Die Wirkung einiger pharmazeutischer Zubereitungen auf die Barrierefunktion der Haut besteht sogar in einer selektiven Barriereschädigung, die ermöglichen soll, daß Wirkstoffe in bzw. durch die Haut in den Körper eindringen können. Ein gestörtes Erscheinungsbild der Haut wird dabei als Nebenwirkung teilweise billigend in Kauf genommen.

Die Wirkung von pflegenden Reinigungsprodukten besteht im wesentlichen in einer effizienten Rückfettung mit Sebumlipid-ähnlichen Substanzen. Durch die gleichzeitige Verminderung des Tensidgehalts solcher Zubereitungen läßt sich der Schaden an der Hornschichtbarriere weiter begrenzen.

Dem Stand der Technik mangelt es allerdings an Zubereitungen, welche die Barrierefunktion und die Hydratation der Hornschicht positiv beeinflussen und die physikalischchemischen Eigenschaften der Hornschicht und insbesondere der Lamellen aus Interzellularlipiden starken bzw. sogar wiederherstellen.

Um die defizitäre Haut bei ihrer natürlichen Regeneration zu unterstützen und ihre physiologische Funktion zu stärken, werden den topischen Präpraten in neuerer Zeit zunehmend Interzellularlipidmischungen zugesetzt, die von der Haut zum Wiederaufbau der natürlichen Barriere verwendet werden sollen. Allerdings handelt es sich bei diesen Lipiden, insbesondere aber den Ceramiden, um sehr teure Rohstoffe. Zudem ist ihre Wirkung meist sehr viel geringer als erhofft.

Ziel der vorliegenden Erfindung war es somit, Wege zu finden, die Nachteile des Standes der Technik zu vermeiden. Insbesondere sollte die Wirkung der Zubereitungen physiologisch, schnell und nachhaltig sein.

Erfindungsgemäß werden die gestellten Aufgaben gelöst.

Erfindungsgemäß werden diese Ziele z.B. von Hautpflegepräparaten und/oder vorzugsweise pflegenden Hautreinigungspräparaten oder Zubereitungen für die Haare erreicht, die Wirkstoffe enthalten, welche die NO-Konzentration oder die Konzentration an NO-Radikalen in der Epidermis, insbesondere in den Zellen der Epidermis, insbesondere in den Keratinozyten, oder den Zellen der Haarwurzeln senken oder die Wirkung von NO oder NO-Radikalen inhibieren. Sie erhöhen z.B. vielfach die Syntheserate der Ceramide, insbesondere der Ceramide 1 bis 7. Die Barrierefunktion wird verbessert.

Gegenstand der Erfindung ist die topische Verwendung eines Wirkstoffes oder mehrerer Wirkstoffe, ausgewählt aus der Gruppe der NO oder NO-Radikale bindenden Substanzen und der Gruppe der die NO-Synthese inhibierenden Substanzen und der Gruppe der Inhibitoren der Wirkungen von NO oder NO-Radikalen, zur Verbesserung der Funktion der Permeabilitätsbarriere der Haut und/oder der Struktur oder des Wachstums der Haare.

Mit NO sind Stickstoffmonoxid, aber auch gegebenenfalls Stickstoffmonoxid-Radikale gemeint.

Gegenstand der Erfindung ist auch die Verwendung von kosmetischen oder dermatologischen topischen Zubereitungen mit einem Gehalt eines Wirkstoffes oder mehrerer Wirkstoffe, ausgewählt aus der Gruppe der NO oder NO-Radikale bindenden Substanzen und der Gruppe der die NO-Synthese inhibierenden Substanzen und der Gruppe der Inhibitoren der Wirkungen von NO oder NO-Radikalen, zur Verbesserung der Funktion der Permeabilitätsbarriere der Haut und/oder der Struktur oder des Wachstums der Haare.

Gegenstand der Erfindung ist auch die Verwendung eines Wirkstoffes oder mehrerer Wirkstoffe, ausgewählt aus der Gruppe der NO oder NO-Radikale bindenden Substanzen und der Gruppe der die NO-Synthese inhibierenden Substanzen und der Gruppe der Inhibitoren der Wirkungen von NO oder NO-Radikalen, zur Herstellung einer kosmetischen oder dermatologischen topischen Zubereitung für die Verbesserung der Funktion der Permeabilitätsbarriere der Haut und/oder der Struktur oder des Wachstums der Haare.

Erfindungsgemäße Wirkstoffe sind insbesondere
(A) NO oder NO-Radikale bindende Stoffe, vorzugsweise 2-Phenyl-4,4,5,5-Tetramethylimidazolin-1-Oxyl-3-Oxid [PTlO], Carboxy-PTlO, [2-(4-Carboxyphenyl)-4,4,5,5-tetramethylimidazoline-1-oxyl-3-oxide)], Eisen-Schwefel-Cluster, Metalloporphyrine, Polyamine und Verbindungen mit freien Thiolgruppen, z.B. N-Azetylcystein, Dithiothreitol,
(B) die NO-Synthese inhibierende Stoffe, vorzugsweise NO-Synthase-Inhibitoren [z.B. L-NAME (= Nitroargininmethylester), L-NMMA (= Monomethylarginin), N-Acylsphingosine = Ceramide; Aminoguanidin, 2-Amino-4-Methylpyridin, N^{G}, N^{G}-Dimethyl-L-Arginin, α-Guanidino-glutarsäure, L-N⁵-N-(1-Iminoethyl)ornithin, S-Methylisothiourea, S-Methyl-L-Thiocitrullin, Ölsäure,
(C) Inhibitoren der Expression der induzierbaren NO-Synthase, vorzugsweise Ubichinon Q0, Benzochinone, Dexamethason,
(D) Substanzen, die die Bindung von NO beispielsweise an aktive Zentren von Enzymen oder an andere zelluläre Strukturen vermindern, vorzugsweise Stoffe, die den intrazellulären pH-Wert senken, z.B. schwache Carbonsäuren, vorzugsweise mit pH-Werten zwischen 3 und 5, insbesondere Monocarbonsäuren.
(E) Inhibitoren der NO-stimulierten, zellulären Guanylatcyclase, vorzugsweise Methylenblau.

Bevorzugt werden die folgenden Wirkstoffe:
(A1) NO-bindende oder NO-Radikale bindende Substanzen, insbesondere
   2-Phenyl-4,4,5,5-Tetramethylimidazolin-1-Oxyl-3-Oxid [PTlO] (Calbiochem S.454 # 523350)
   Carboxy-PTlO (Calbiochem S. 454 # 217385)
   Metalloporphyrine
   (+) Rutin-Hydrat (Calbiochem S.478 # 558940)
   Substanzen mit freien Thiolgruppen:
   N-Azetylcystein (Calbiochem S.471 # 106425)
   Dithiothreitol (Sigma S. 396 # D5545)
(B1) NO-Synthese inhibierende Substanzen oder Inhibitoren der Auswirkungen von NO
   N^{G}-Allyl-L-Arginin (Calbiochem S. 440 #128100)
   N^{G}-Nitro-D,L-Argininmethylester, Calb. S. 446/447 # 483124/483125)
   N^{G}-Nitro-D,L-Arginin (Calb. S. 446 # 483121 bzw. 483120)
   N^{G}-Monomethylarginin, Calb. S.445 # 475886)
   N^{g}-Monoethyl-L-Arginin (Calb. S. 445 # 475883)
   N^{g}-Monopropyl-L-Arginin (Calb S. 448 # 537200)
   N^{G}-Monomethyl-L-Homoarginin (Calb. S.446 # 475890)
   Argininderivate
   L-N⁶-(1-Iminoethyl)Lysin (Calb. S.446 # 482100)
   L-N⁵-N-(1-Iminoethyl)ornithin (Calb. S.444 # 400600)
   Epigallocatechin-Gallat (Calbiochem S.474 # 324880)
   Na-Salicylat (Calb. S. 449 #567630)
   C₂-C₃₂-N-Acylsphingosin= Ceramid
   Spermidin (Calb. S.449 # 56766 und andere Polyamine)
   Spermin (Calb. S.402 # 5677)
   Aminoguanidin (Calbiochem S. 440 # 154500)
   2-Amino-4-Methylpyridin (Calb. S.441 #164575)
   N^{G},N^{G}-Dimethyl-L-Arginin (Calb. S. 443 # 311204)
   S-Methylisothiourea (Calb. S.444 # 466220)
   S-Methyl-L-Thiocitrullin (Calb. S. 444 # 472804)
   Ölsäure
   Melatonin (Calb. S. 153 # 444300)
   Ubichinon Q0 (=2,3-Dimethoxy-5-methyl-benzochinon)
   Benzochinone
   Zink (II) Protoporphyrin IX (Calb. S. 453 # 691550)
   Methylenblau (Calb. S. 452 # 457250)

In Klammern sind jeweils Seitenzahlen und Produktnummern der Wirkstoffe angegeben, wie sie im Katalog 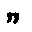 Signal Transduction Catalog & Technical Resource 1999" der Firma Calbiochem-Novabiochem GmbH, Postfach 1167, D-65796 Bad Soden/Ts. beschrieben sind.

Weitere geeignete NO-Synthase-Hemmer sind beispielsweise
2-Iminobiotin,
L-N⁵-(1-Iminoethyl)-ornithin (L-NIO),
S-Methylisothioharnstoff
S-Methylisothioharnstoff-sulfat (SMT),
S-Methyl-L-thiocitrullin,
L-N^{G}-(1-Iminoethyl)-Iysin(L-NIL),
7-Nitroindazol (7-Ni),
S,S'-1,3-Phenylen-bis-(1,2-ethan-di-yl)-bis-isothioharnstoff (PBITU)
L-Thiocitrullin (2-Thioureido-L-norvaline)
und deren Derivate und insbesondere die Argininderivate.

Bevorzugt werden NO-Synthase-Hemmer, die eine Guanidingruppe enthalten.

Geeignete Derivate sind beispielsweise die an den Iminogruppen oder Aminogruppen monoalkylierten oder dialkylierten erfindungsgemäßen Verbindungen.

Jeweils können die Alkylreste der Monoalkylgruppen oder Dialkylgruppen 1 bis 10, vorzugsweise 1 bis 6, insbesondere aber 1, 2 oder 3 Kohlenstoffatome besitzen und geradkettig oder verzweigt sein.

Gut geeignet sind auch Derivate, insbesondere des Arginins, deren Aminogruppen vollständig oder teilweise acyliert sind. Es sind dies insbesondere die Aminogruppen des Aminosäurerestes und insbesondere die an das alpha-C-Atom gebundenen Aminogruppen. Bevorzugt werden die mono-Acyl -Verbindungen des erfindungsgemäßen Wirkstoffes, insbesondere eines Argininderivates.

Bevorzugter Acylrest ist Alkylcarbonyl, der bei Acylierungen mit Carbonsäuren bzw. deren Derivaten, z.B. Säurechloriden oder Anhydriden, erhalten wird. Der Acylrest bzw. Alkylcarbonylrest kann 2 - 12, insbesondere 2 - 6 Kohlenstoffatome besitzen und ist besonders bevorzugt Acetyl.

Die Verbindung alpha-N-Acetyl-N^{G}-nitro-L-arginin-methylester (alpha-N-Acetyl-L-NAME), in der also die Aminogruppe des alpha-C-Atoms der Aminosäurefunktion mono-acetyliert ist, wird besonders bevorzugt.

Die Acylderivate zeichnen sich bei guter Wirksamkeit durch die Lagerstabilität und ihre Stabilitat in den Zubereitungen aus.

Geeignete Derivate der erfindungsgemäßen Verbindungen sind insbesondere die Salze und Säureadditionssalze. Auch Ester von Carbonsäuregruppen der erfindungsgemäßen Verbindungen mit Alkoholen sind bevorzugt.

Bevorzugte Salze sind wasserlösliche Salze, z.B. Natrium-, Kalium- und Ammoniumsalze. Dies gilt auch für die Säureadditionssalze. Geeignete Säureadditionssalze werden z.B. mit anorganischen und organischen Säuren erhalten. Bevorzugt werden die Hydrochloride, Phosphate, Sulfate, Acetate, Caprylate, Zitrate, Lactate, Malate oder Tartrate.

Geeignete Ester sind z.B. solche, die mit kurzkettigen oder mittelkettigen Alkoholen gebildet werden, vorzugsweise mit mono-Alkoholen. Sie können geradkettig oder verzweigt sein und z.B. 1 bis 12, vorzugsweise 1 bis 6 Kohlenstoffatome besitzen. Bevorzugt werden Methanol, Ethanol, n-Propanol und iso-Propanol.

Die Ester sind besonders bevorzugte Derivate. Sie zeichnen sich z.B. auch durch eine bessere Penetration aus.

Die erfindungsgemäßen Verbindungen sind an sich bekannt, im Handel erhältlich oder können nach bekannten Verfahren erhalten werden. In der Literatur ist ihre Wirkung als NO-Synthase-Hemmer beschrieben. Die acylierten Verbindungen können mit den bekannten Acylierungsverfahren erhalten werden.

Besonders bevorzugt werden erfindungsgemäße NO-Synthase-Hemmer, die einen Argininrest enthalten und deren Derivate, insbesondere wie im folgenden beschrieben.

Bevorzugt werden eine Verbindung oder mehrere Verbindungen ausgewählt aus der Gruppe von N^{G}-Monoalkyl-L-Arginin, N^{G}, N^{G}-Dialkyl-L-arginin, N^{G}, N^{G'}-Dialkyl-L-arginin und N^{G}-Nitro-L-Arginin und deren Derivate.

Jeweils können die Alkylreste der Monoalkylgruppen oder Dialkylgruppen 1 bis 10, vorzugsweise 1 bis 6, insbesondere aber 1, 2 oder 3 Kohlenstoffatome besitzen und geradkettig oder verzweigt sein.

Geeignete Derivate der erfindungsgemäßen Verbindungen sind insbesondere die Salze und Säureadditionssalze. Auch Ester der Carbonsäuregruppe des Arginins mit Alkoholen sind besonders bevorzugt.

Bevorzugte Salze sind wasserlösliche Salze, z.B. Natrium-, Kalium- und Ammoniumsalze. Dies gilt auch für die Säureadditionssalze. Geeignete Säureadditionssalze werden z.B. mit anorganischen und organischen Säuren erhalten. Bevorzugt werden die Hydrochloride, Phosphate, Sulfate, Acetate, Caprylate, Zitrate, Lactate, Malate oder Tartrate.

Geeignete Ester sind z.B. solche, die mit kurzkettigen oder mittelkettigen Alkoholen gebildet werden, vorzugsweise mit mono-Alkoholen. Sie können geradkettig oder verzweigt sein und z.B. 1 bis 12, vorzugsweise 1 bis 6 Kohlenstoffatome besitzen. Bevorzugt werden Methanol, Ethanol, n-Propanol und iso-Propanol.

Die Ester sind besonders bevorzugte Derivate. Sie zeichnen sich auch durch eine bessere Penetration aus.

Bevorzugt werden die folgenden Verbindungen:
N^{G}-Monomethyl-L-arginin,
N^{G}-Monoethyl-L-arginin,
N^{G}-Nitro-L-arginin,
N^{G}-Nitro-L-arginin-methylester,
N^{G}-Nitro-L-arginin-ethylester,
N^{G}-Monomethyl-L-arginin-methylester,
N^{G}-Monoethyl-L-arginin-methylester,
N^{G}-Monomethyl-L-arginin-ethylester
N^{G}-Monoethyl-L-arginin-ethylester und
N^{G}, N^{G}-Dimethyl-L-arginin,
N^{G}, N^{G'}-Dimethyl-arginin
N^{G}, N^{G}-Dimethyl-L-arginin-dihydrochlorid,
N^{G}, N^{G'}-Dimethyl-L-arginin-dihydrochlorid

Besonders bevorzugt werden die folgenden Verbindungen:
N^{G}-Monomethyl-L-arginin-monoacetat (L-NMMA),
N^{G}-Monoethyl-L-arginin-monoacetat (L-MEA),
N^{G}₋Nitro-L-arginin (L-NNA), N^{G}₋Nitro-D, L-arginin,
N^{G}-Nitro-L-arginin-methylester-hydrochlorid(L-NAME).
N^{G}-Nitro-L-arginin-methylester oder
L-NAME wird ganz besonders bevorzugt.

Die erfindungsgemäßen dermatologischen und kosmetischen topischen Zubereitungen können als Wirkstoff einen NO-Synthase-Hemmer oder mehrere NO-Synthase-Hemmer enthalten, z.B. eine, zwei oder drei Verbindungen.

Die erfindungsgemäßen Wirkstoffe können in ihren optisch aktiven Formen oder als Racemate verwendet werden.

Vorteilhaft enthalten erfindungsgemäße Zubereitungen 0,00001 - 20 Gewichtsprozente der erfindungsgemäßen Substanzen bzw. Substanzklassen, wobei die Substanzen allein oder in Kombination enthalten sein können.

Bevorzugt enthalten die Zubereitungen 0,0001 - 10 Gew.-% der erfindungsgemäßen erfindungsgemäßen Wirkstoffe.

Ganz besonders bevorzugt enthalten die Zubereitungen 0,001 - 1 Gew.-% der erfindungsgemäßen Wirkstoffe.

Im Rahmen der Anmeldung sind Gewichtsprozente bezogen auf 100% Gesamtzusammensetzung des jeweiligen erfindungsgemäßen Kosmetikums oder Dermatikums gemeint.

Durch die erfindungsgemäßen Wirkstoffe und die kosmetischen oder dermatologischen topischen Zubereitungen, die diese Wirkstoffe enthalten, werden insbesondere die folgenden Mängel und Störungen behoben oder zumindest wesentlich gebessert:

Trockene Haut in jungen Jahren und im Alter, insbesondere aber die nicht-ekzematöse Haut von Atopikern, neigt zur Hyperproliferation und dysregulierten epidermalen Differenzierung und weist generell einen Mangel an Ceramiden in der Hornschicht auf. Dieser Mangel an Sphingolipiden tragt z.B. zur Schwäche der Permeabilitätsbarriere aus multilamellaren Interzellularmembranen in der Hornschicht der Haut bei und ist somit wahrscheinlich ursächlich für die Hauttrockenheit, z.B. direkt (über Ceramid-abhängige Signalwege) oder indirekt (über den erhöhten TEWL) für die.erhöhte Proliferationsrate und gestörte Zellreifung, z.B. bei Neurodermitis.

Die erfindungsgemäßen Wirkstoffe und Zubereitungen sind auch zur Behandlung und prophylaktischen Behandlung der Neurodermitis oder der nicht-ekzematösen Haut des Atopikers geeignet.

Erfindungsgemäße topische Zubereitungen oder Zusammensetzungen mit den erfindungsgemäßen Kombinationen und Wirkstoffen sind alle gängigen Anwendungsformen, z.B. Cremes (W/O, O/W, W/O/W), Gele, Lotionen, Milchen.

Kosmetische Zubereitungen werden bevorzugt.

Die erfindungsgemäßen topischen Zubereitungen können als flüssige, pastöse oder feste Zubereitungen formuliert werden, beispielsweise als wäßrige oder alkoholische Lösungen, wäßrige Suspensionen, Emulsionen, Salben, Cremes, Öle, Pulver oder Stifte. In Abhängigkeit von der gewünschten Formulierung können Wirkstoffe in pharmazeutische und kosmetische Grundlagen für topische Applikationen eingearbeitet werden, die als weitere Komponenten beispielsweise Ölkomponenten, Fell und Wachse, Emulgatoren, anionische, kationische, ampholytische, zwitterionische und/oder nichtionogene Tenside, niedere ein- und mehrwertige Alkohole, Wasser, Konservierungsmittel, Puffersubstanzen, Verdickungsmittel, Duftstoffe, Farbstoffe und Trübungsmittel enthalten. Vorteilhaft können die erfindungsgemäßen Wirkstoffe auch in transdermalen therapeutischen Systemen, insbesondere kubischen Systemen verwendet werden, oder auch in Haarbehandlungsmitteln oder Haarreinigungsmitteln, wobei die Wirkstoffe z.B. an der Kopfhaut oder in der Haarwurzel ihre Wirkung entfalten.

Die Gesamtmenge der vorstehend genannten Stoffe oder Wasser beträgt beispielsweise 1 bis 99 Gew.-%, jeweils bezogen auf das Gesamtgewicht der Zubereitung.

Die erfindungsgemäßen Wirkstoffe und Zubereitungen können weiterhin auch zur Behandlung der Haare dienen. Sie verbessern z.B. die Haarstruktur und können das Wachstum der Haare fördern und Haarausfall vermindern. Geeignete Zubereitungen sind z.B. die bekannten Haarbehandlungs- oder Haarreinigungsmittel, denen die Wirkstoffe zugesetzt werden können.

Die Anwendung der erfindungsgemäßen Zubereitungen kann in der für topische Zubereitungen üblichen Weise, z.B. einmal oder mehrmals täglich erfolgen und wird vorzugsweise über einen gewissen Zeitraum, z.B. mehrere Tage oder auch mehrere Wochen vorgenommen, so daß sich eine gewünschte höhere Ceramidkonzentration in der Hornschicht aufbauen kann.

Es ist ferner von Vorteil, den topischen Zubereitungen Zusatzstoffe wie Vitamine, Coenzyme, Substrate und Hilfsfaktoren des Lipidstoffwechsels (z.B. Pyridoxin, Pyridoxal, Pyridoxamin, Uridin, L-Serin, schwache Carbonsäuren, deren pKs-Wert zwischen 3 und 5,5 liegt wie z.B. Milchsäure und Propionsäure), des Energiestoffwechsels (z.B. Zitronensäure, Pyruvat sowie zelluläre Energieüberträger [z.B. Kreatin, Guanin, Guanosin, Adenin, Adenosin, Nicotin, Nicotinamid, Riboflavin], Coenzyme [z.B. Coenzym Q10, Pantothensäure, Panthenol, Liponsäure], Hilfafaktoren [z.B. L-Carnitin], Substrate [z.B. Hexosen, Pentosen, Fettsäuren]); Taurocholsäure; Lipide (z.B. Ceramide, Cholesterin, Fettsäuren, Sphinosin, Sphingomyelin, Glucocerebroside), Substrate (z.B. Hexosen, Pentosen, Fettsäuren), Glutathion und/oder natürliche Befeuchtungsfaktoren (z.B. Aminosäuren, Harnstoff, Pyrrolidoncarbonsäure, Glycerin) zuzusetzen.

Ganz besonders vorteilhaft ist es, den Zubereitungen ein- oder mehrfach cis-ungesättigte Fettsäuren wie z.B. Ölsäure, Linolsäure, Gammalinolensäure, Arachidonsäure und/oder Katechine ((-)-Catechin, (+)-Catechin, (-)-Catechin Gallat, (-)-Gallocatechin Gallat, (+)-Epicatechin, (-)-Epicatechin, (-)-Epicatechin Gallat, (-)-Epigallocatechin, (-)-Epigallocatechin Gallat) oder Extrakte des grünen Tees hinzuzufügen.

Darüber hinaus enthalten die Zubereitungen sinnhafterweise freie Sterole (z.B. Lanosterol, Cholesterol), andere, mittellange bis lange, gesättigte Fettsäuren (Stearinsäure, Palmitinsäure, Lignocerinsäure) und Ceramide (allgemein N-Acyl-Phytosphingosin, N-Acyl-Sphingosin oder N-Acyl-6-Hydroxy-4-Sphingenin).

Die Gesamtmenge der jeweiligen, vorstehend genannten Stoffe beträgt beispielsweise 0,0001 bis 20,0 Gew.-%, insbesondere 0,001 bis 10 Gew.-%, jeweils bezogen auf das Gesamtgewicht der Zubereitung.

Es ist auch von Vorteil, den Zubereitungen Antioxidantien (z.B. alpha-Tocopherol, Vitamin E und C, Imidazole, alpha-Hydroxycarbonsäuren (z.B. Apfelsäure, Glycolsäure, Gluconsäure, Salicylsäure sowie deren Derivate) und/oder Eisenkomplexbildner (z.B. EDTA, alpha-Hydroxyfettsäuren) und/oder bekannte UV-Lichtschutzfilter in Mengen von z.B. **0,01** bis 10 Gew.-% zuzusetzen, um die Stabilität der oxidationsempfindlichen Wirkstoffe zu gewährleisten.

Vorteilhaft können erfindungsgemäße Zubereitungen außerdem Substanzen enthalten, die UV-Strahlung im UVA- und/oder im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1,0 bis 6,0 Gew.-% beträgt, bezogen auf das Gesamtgewicht der Zubereitungen, um kosmetische Zubereitungen zur Verfügung zu stellen, die die Haut vor dem gesamten Bereich der ultravioletten Strahlung schützen. Sie können auch als Sonnenschutzmittel für die Haut dienen. In den Zubereitungen wirken die UV-Absorber gegenüber den Wirkstoffen als Antioxidantien.

Enthalten die erfindungsgemäßen Emulsionen UVB-Filtersubstanzen, können diese öllöslich oder wasserlöslich sein. Erfindungsgemäß vorteilhafte öllösliche UVB-Filter sind z.B.: 3-Benzylidencampher-Derivate, vorzugsweise 3-(4-Methylbenzyliden)campher, 3-Benzylidencampher.

Vorteilhaft wasserlösliche UVB-Filter sind z.B.:

Salze der 2-Phenylbenzimidazol-5-sulfonsäure wie ihr Natrium-, Kalium- oder ihr Triethanolammonium-Salz, sowie die Sulfonsäure selbst.

Es kann auch von Vorteil sein, erfindungsgemäße Wirkstoffkombinationen mit UVA-Filtern zu kombinieren, die bisher üblicherweise in kosmetischen Zubereitungen enthalten sind. Bei diesen Substanzen handelt es sich vorzugsweise um Derivate des Dibenzoylmethans, insbesondere um 1-(4'-tert.Buthylpheny)-3(4'-methoxyphenyl)-propan-1,3-dion und um 1-Phenyl-3-(4'isopropylphenyl)propan-1, 3dion. Auch diese Kombinationen bzw. Zubereitungen, die diese Kombinationen enthalten, sind Gegenstand der Erfindung. Es können die für die UVB-Kombination verwendeten Mengen eingesetzt werden.

Gegenstand der Erfindung sind also auch die Kombinationen der erfindungsgemäßen Wirkstoffe, insbesondere in den topischen Zubereitungen, mit Antioxidantien, Stoffen des aeroben zellulären Energiestoffwechsels und/oder UV-Absorbern, durch die sich z.B. die Stabilität und die Wirkung der Zubereitung verbessern läßt.

Die vorstehend aufgeführten Beispiele für kombinierbare Wirkstoffe aus den angegebenen Wirkstoffgruppen dienen dazu, die Erfindung zu beschreiben, ohne daß beabsichtigt ist, die Erfindung auf diese Beispiele zu beschränken.

Darüber hinaus können schützende Formulierungsformen angewendet werden, wobei die erfindungsgemäßen Stoffe z.B. in Liposomen, Micellen, Nanosphären usw. aus z.B. hydrierten Amphiphilen, wie z. B. Ceramiden, Fettsäuren, Sphingomyelin und Phosphoglyceriden bzw. in Zyklodextrane eingeschlossen (verkapselt) werden. Weiterer Schutz kann durch die Verwendung von Schutzgas (z.B. N₂, CO₂) bei der Formulierung und die Verwendung gasdichter Verpackungsformen erreicht werden.

Weitere Hilfs- und Zusatzstoffe können wasserbindende Stoffe, Verdicker, Füllstoffe, Parfüm, Farbstoffe, Emulgatoren, Wirkstoffe wie Vitamine, Konservierungsmittel, Wasser und/oder Salze sein, z.B. in den vorstehend schon genannten Mengen.

Bei der Verarbeitung der Wirkstoffe und anderer oxidationsempfindlicher Stoffe sollte die Temperatur nicht über 40°C liegen. Ansonsten sind die üblichen Maßregeln zu beachten, die dem Fachmann bekannt sind. Die Herstellung der Zubereitungen kann in an sich bekannter Weise durch Mischen oder Emulgieren der Bestandteile erfolgen.

Die erfindungsgemäßen Stoffgruppen lassen sich so in kosmetischen Grundlagen einarbeiten. Grundsätzlich sind allerdings W/O- und O/W- und W/O/W-Emulsionen bevorzugt. Besonders vorteilhaft können erfindungsgemäße Kombinationen in Pflegeprodukte wie beispielsweise O/W-Cremes, W/O-Cremes, O/W-Lotionen usw. eingesetzt werden.

Mengenangaben, Prozentangaben oder Teile beziehen sich, soweit nicht anders angegebene, auf das Gewicht, insbesondere auf das Gesamtgewicht der Zubereitungen oder der jeweiligen Mischung.

Die folgenden Beispiele dienen nur dazu, die Erfindung zu beschreiben, ohne daß beabsichtigt ist, die Erfindung auf diese Beispiele zu schränken. Die Mengenangaben sind Gewichtsteile oder Gewichtsprozent bei den 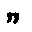 100" Angaben.

### Beispiel I

### Hautcreme vom W/O-Typ

| | |
|---|---|
| Vaseline DAB 9 | 13 |
| Glycerin DAB 9 | 6,3 |
| Wasser VES | 34,4 |
| Paraffinöl (Mineralöl 5E,Shell) | 40,8 |
| Cetearylalkohol/TEG-40-Castor Oil/Natriumcetearyl-sulfat (Eutanol G, Henkel KGaA) | 2,5 |

3 Teile Carboxy-PTlO werden in die Wasserphase, bestehend aus Wasser und Glycerin, eingerührt. Die Fettphase wird gemischt und zu der 75°C warmen Wasserphase gegeben, verrührt und homogenisiert, bis eine gleichmäßige Creme entstanden ist.

Beispiel I hat folgende endgültige Zusammensetzung:

| | Gew.-% |
|---|---|
| Vaseline DAB 9 | 13 |
| Glycerin DAB 9 | 6,3 |
| Wasser VES | 34,4 |
| Paraffinöl (Mineralöl 5E,Shell) | 40,8 |
| Cetearylalkohol/TEG-40-Castor Oil/Natriumcetearyl-sulfat (Eutanol G, Henkel KGaA) | 2,5 |
| Carboxy-PTlO, [2-(4-Carboxyphenyl)-4,4,5,5-tetramethylimidazoline-1-oxyl-3-oxide)] | 3,0 |

### Beispiel II

### Hautcreme vom W/O-Typ

| | |
|---|---|
| PEG-1 Glyceryl-Oleostearat + Paraffinwachs | 8 |
| Vaseline DAB | 2,8 |
| Paraffinwachs/Paraffin | 1,8 |
| Paraffinöl (Mineralöl 5E,Shell) | 12 |
| Ceresin | 2,2 |
| Octyldodecanol | 10 |
| Propylenglycol | 1 |
| Glycerin | 1 |
| Magnesiumsulfat | 0,7 |
| Wasser VES | 58,7 |
| Summe Additive (Parfüm, Konservierung, Stabilisation) | 0,8 |

1 Teil L-NAME wird in der Wasserphase, bestehend aus Wasser und Glycerin, gelöst. Die Fettphase wird gemischt und zu der 75°C warmen Wasserphase gegeben, verrührt und homogenisiert, bis eine gleichmäßige Creme entstanden ist.

Beispiel II hat folgende endgültige Zusammensetzung:

| PEG-1 Glyceryl-Oleostearat + | |
|---|---|
| | Gew.-% |
| Paraffinwachs | 8 |
| Vaseline DAB | 2,8 |
| Paraffinwachs/Paraffin | 1,8 |
| Paraffinöl (Mineralöl 5E,Shell) | 12 |
| Ceresin | 2,2 |
| Octyldodecanol | 10 |
| Propylenglycol | 1 |
| Glycerin | 1 |
| Magnesiumsulfat | 0,7 |
| Wasser VES | 58,7 |
| L-NAME | 1 |
| Summe Additive (Parfüm, Konservierung, Stabilisation) | 0,8 |

### Beispiel III

### Hautcreme vom O/W-Typ

| | |
|---|---|
| Octyldodecanol (Emulgade F, Henkel KGaA) | 9,3 |
| Cetearylalkohol/TEG-40-Paraffinöl (Mineralöl eE, Shell) | 7,8 |
| Castor Oil/Natriumcetearyl-sulfat (Eutanol G, Henkel KGaA) | 3,7 |
| Wasser VES | 73,7 |
| Glycerin DAB 9 | 4,5 |

1 Teil Ubichinon Q0 (=2,3-Dimethoxy-5-methyl-benzochinon) wird in der Wasserphase, bestehend aus Wasser und Glycerin, gelöst. Die Fettphase wird gemischt und zu der 75°C warmen Wasserphase gegeben, verrührt und homogenisiert, bis eine gleichmäßige Creme entstanden ist.

Beispiel III hat folgende endgültige Zusammensetzung:

| | Gew.-% |
|---|---|
| Octyldodecanol (Emulgade F, Henkel KGaA) | 9,3 |
| Cetearylalkohol/TEG-40-Paraffinöl (Mineralöl eE, Shell) | 7,8 |
| Castor Oil/Natriumcetearyl-sulfat (Eutanol G, Henkel KGaA) | 3,7 |
| Wasser VES | 73,7 |
| Glycerin DAB 9 | 4,5 |
| Ubichinon Q₀ | 1,0 |

### Beispiel IV

### O/W-Lotion

| | |
|---|---|
| Steareth-2 | 3 |
| Steareth-21 | 2 |
| Cetearylalkohol/TEG-40-Castor Oil/Natriumcetearyl-sulfat (Eutanol G, Henkel KGaA) | 2,5 |
| Paraffinöl (Mineralöl eE, Shell) | 15 |
| Propylenglycol | 1 |
| Glycerin | 3 |
| Wasser VES | 71,5 |
| Summe Additive (Parfüm, Konservierung, Stabilisation) | 0,8 |

0,6 Teile (-)-Catechin und 0,6 Teile L-NMMA werden in der Wasserphase, bestehend aus Wasser und Glycerin, gelöst. Die Fettphase wird gemischt und sodann zu der 75°C warmen Wasserphase gegeben, verrührt und homogenisiert, bis eine gleichmäßige hellgelbe Lotion entstanden ist.

Beispiel IV hat folgende endgültige Zusammensetzung:

| | Gew.-% |
|---|---|
| Steareth-2 | 3 |
| Steareth-21 | 2 |
| Cetearylalkohol/TEG-40-Castor Oil/Natriumcetearyl-sulfat (Eutanol G, Henkel KGaA) | 2,5 |
| Paraffinöl (Mineralöl eE, Shell) | 15 |
| Propylenglycol | 1 |
| Glycerin | 3 |
| Wasser VES | 71,5 |
| Summe Additive (Parfüm, (-)-Catechin | 0,6 |
| L-NMMA | 0,6 |
| Konservierung, Stabilisation) | 0,8 |

### Beispiel V

### O/W-Lotion

| | |
|---|---|
| Octyldodecanol (Emulgade F, Henkel KGaA) | 5,6 |
| Cetearylalkohol/TEG-40-Castor Oil/Natriumcetearyl-sulfat (Eutanol G, Henkel KGaA) | 8,9 |
| Cetearylisononanoat (Cetiol 5N, Henkel KGaA) | 7,5 |
| Paraffinöl (Mineralöl eE, Shell) | 10,5 |
| Wasser VES | 57,8 |
| Glycerin DAB 9 | 4,7 |

1 Teil L-NAME und 4 Teile Carboxy-PTlO werden in der Wasserphase, bestehend aus Wasser und Glycerin, gelöst, Die Fettphase wird gemischt und sodann zu der 75°C warmen Wasserphase gegeben, verrührt und homogenisiert, bis eine gleichmäßige Lotion entstanden ist.

Beispiel V hat folgende endgültige Zusammensetzung:

| | Gew.-% |
|---|---|
| Octyldodecanol (Emulgade F, Henkel KGaA) | 5,6 |
| Cetearylalkohol/TEG-40-Castor Oil/Natriumcetearyl-sulfat (Eutanol G, Henkel KGaA) | 8,9 |
| Cetearylisononanoat (Cetiol 5N, Henkel KGaA) | 7,5 |
| Paraffinöl (Mineralöl eE, Shell) | 10,5 |
| Wasser VES | 57,8 |
| L-NAME | 1,0 |
| Carboxy-PTlO | 4,0 |
| Glycerin DAB 9 | 4,7 |

### Beispiel VI

### Hautöl

| | |
|---|---|
| Glyceryltricaprylat (Miglyol 812, Dynamit Nobel) | 21 |
| Hexyllaurat (Cetiol A, Henkel KGaA) | 20 |
| Octylstearat (Cetiol 886, Henkel KGaA | 20 |
| Paraffinöl (Mineralöl 5E, Shell) | 36 |
| Ubichinon Q₀ | 2,7 |
| Ölsäure | 0,3 |

Die Komponenten werden bei 25°C verrührt, bis eine gleichmäßige, klare Mischung entstanden ist.

Ansonsten sind die üblichen Maßregeln zu beachten, die dem Fachmann bekannt sind.

## Patentansprüche

1. Topische Verwendung eines Wirkstoffes oder mehrerer Wirkstoffe, ausgewählt aus der Gruppe der NO oder NO-Radikale bindenden Substanzen und der Gruppe der die NO-Synthese inhibierenden Substanzen und der Gruppe der Inhibitoren der Wirkungen von NO oder NO-Radikalen, zur Verbesserung der Funktion der Permeabilitätsbarriere der Haut und/oder der Verbesserung der Struktur oder des Wachstums der Haare.

2. Verwendung von kosmetischen oder dermatologisch topischen Zubereitungen mit einem Gehalt eines Wirkstoffes oder mehrerer Wirkstoffe, ausgewählt aus der Gruppe der NO oder NO-Radikale bindenden Substanzen und der Gruppe der die NO-Synthese inhibierenden Substanzen und der Gruppe der inhibitoren der Wirkungen von NO oder NO-Radikalen, zur Verbesserung der Funktion der Permeabilitätsbarriere der Haut und/oder der Verbesserung der Struktur oder des Wachstums der Haare.

3. Verwendung eines Wirkstoffes oder mehrerer Wirkstoffe, ausgewählt aus der Gruppe der NO oder NO-Radikale bindenden Substanzen und der Gruppe der die NO-Synthese inhibierenden Substanzen und der Gruppe der Inhibitoren der Wirkungen von NO oder NO-Radikalen, zur Herstellung einer kosmetischen oder dermatologischen topischen Zubereitung für die Verbesserung der Funktion der Permeabilitätsbarriere der Haut und/oder der Verbesserung der Struktur oder des Wachstums der Haare.

4. Verwendung von kosmetischen oder dermatologisch topischen Zubereitungen mit einem Gehalt eines Wirkstoffes oder mehrerer Wirkstoffe, ausgewählt aus der Gruppe der NO oder NO-Radikale bindenden Substanzen und der Gruppe der die NO-Synthese inhibierenden Substanzen und der Gruppe der Inhibitoren der Wirkungen von NO oder NO-Radikalen, oder die Verwendung dieser Wirkstoffe, zur Pflege und Reinigung trockener und beanspruchter Haut und Altershaut sowie zur Behandlung und Prophylaxe der Hautaustrockung und der Beanspruchung der Haut sowie jeweils deren Folgeschäden.

5. Zubereitungen oder Verwendungen gemäß den Ansprüchen 1-4, dadurch gekennzeichnet, daß die Wirkstoffmenge 0,00001 bis 20 Gew.-%, bezogen auf das Gesamtgewicht der Zubereitung, beträgt.

6. Zubereitungen oder Verwendungen gemäß den Ansprüchen 1-5, dadurch gekennzeichnet, daß als Wirkstoffe NO-Synthase-Inhibitoren verwendet werden.

7. Zubereitungen oder Verwendungen gemäß den Ansprüchen 1-6, dadurch gekennzeichnet, daß als Wirkstoff N^{G}-Nitro-arginin verwendet wird.

8. Verwendung von kosmetischen oder dermatologisch topischen Zubereitungen mit einem Gehalt eines Wirkstoffes oder mehrerer Wirkstoffe, ausgewählt aus der Gruppe der NO oder NO-Radikale bindenden Substanzen und der Gruppe der die NO-Synthese inhibierenden Substanzen und der Gruppe der Inhibitoren der Wirkungen von NO oder NO-Radikalen, oder die Verwendung dieser Wirkstoffe, zur Behandlung oder prophylaktischen Behandlung der Neurodermitis oder der nicht-ekzematösen Haut des Atopikers.
